# FASCICULE DE BREVET EUROPEEN

(11) **EP 1 123 692 B1**
(45) Date de publication et mention de la délivrance du brevet: **05.04.2006**
(21) Numéro de dépôt: 00403596.0
(22) Date de dépôt: 19.12.2000
(51) Int. Cl.: A61K 8/44, A61Q 5/02, A61Q 19/10, A61P 17/10

(54) **Utilisation de dérivés de polyaminoacides pour traiter la séborrhée et les désordres cutanés associés**
Verwendung von Polyaminosäurederivaten zur Behandlung von Seborrhoe und verwandten Hautstörungen
Use of polyaminoacid derivatives for the treatment of Seborrhea and related cutaneous disorders

(30) Priorité: 28.01.2000 FR 0001210
(43) Date de publication de la demande: 16.08.2001
(73) Titulaire: L'OREAL, 75008 Paris (FR)
(72) Inventeur: Philippe, Michel, 91320 Wissous (FR); Cupferman, Sylvie, 94240 L'Hay Les Roses (FR)
(74) Mandataire: Le Blainvaux Bellegarde, Françoise

(56) Documents cités:
- WO-A-98/10743
- DE-A- 19 600 480
- DE-C- 4 103 275
- FR-A- 2 718 028
- FR-A- 2 776 510
- US-A- 4 147 782
- US-A- 4 472 382
- US-A- 5 124 313
- US-A- 5 681 586
- MORELLE J: "LIPOAMINOACIDES ET COSMETOLOGIE" PARFUMS COSMETIQUES SAVONS DE FRANCE,FR,PARIS, vol. 3, no. 2, 1 février 1973 (1973-02-01), pages 82-93, XP000106572
- PATENT ABSTRACTS OF JAPAN vol. 1998, no. 14, 31 décembre 1998 (1998-12-31) & JP 10 245396 A (POLA CHEM IND INC), 14 septembre 1998 (1998-09-14)

## Description

La présente invention a pour objet l'utilisation de dérivés de polyaminoacides, dans une composition cosmétique ou pharmaceutique, notamment dermatologique, pour traiter la séborrhée et les désordres cutanés qui lui sont associés.

La sécrétion de sébum est un phénomène normal et utile à la peau comme à la chevelure. Cependant, l'hypersécrétion de sébum, appelée la séborrhée, entraîne des désagréments et parfois une pathologie cutanée, notamment une peau grasse et même acnéique, et un état séborrhéique du cuir chevelu. L'hypersécrétion sébacée et la perturbation de la kératinisation du follicule pilo-sébacé ont pour conséquences l'obstruction du follicule pilo-sébacé et la formation de lésions rétentionnelles ou comédons.
Ces désordres cutanés, et en particulier l'acné et/ou l'hyperséborrhée, relèvent notamment de la colonisation de la peau ou du follicule pileux par des germes du genre *Propionibacterium* tels que *Propionibacterium acnes, Propionibacterium granulosum* et *Propionibacterium avidum.*
Pour lutter contre ces agents pathogènes, on utilise couramment des actifs tels que le triclosan, l'hexamidine, l'hexétidine et le chlorure de benzalkonium. Mais l'utilisation de ces actifs n'est pas dénuée d'effets secondaires. Ainsi, le triclosan présente une toxicité non négligeable, même par voie topique. En outre, il s'est révélé insuffisamment efficace, notamment dans certains véhicules où son activité est inhibée par les tensioactifs. L'hexamidine et l'hexétidine sous forme de sels sont des substances sensibilisantes, susceptibles de provoquer des allergies. Par ailleurs, le chlorure de benzalkonium peut se révéler irritant aux doses habituelles d'utilisation. De plus, il déstabilise des compositions contenant des tensioactifs anioniques.
On constate donc que subsiste le besoin d'actifs topiques ayant un effet sur les pathologies liées aux germes du genre *Propionibacterium,* ayant une action au moins aussi efficace que les composés de l'art antérieur, tout en ne présentant pas les inconvénients des composés connus.
La présente invention a pour but de proposer de nouveaux composés particuliers permettant d'obtenir cet effet.

L'invention a donc pour objet l'utilisation d'au moins un dérivé de polyaminoacide de formule (I) tel que ci-après défini, ou d'un de ses sels,
- pour traiter cosmétiquement la séborrhée de la peau et/ou du cuir chevelu, et/ou les désordres cutanés associés à la séborrhée, et/ou les désordres liés aux germes du genre *Propionibacterium,* notamment *Propionibacterium acnes* et/ou Pro*pionibacterium granulosum;* ou
- pour la fabrication d'une composition pharmaceutique destinée à traiter la séborrhée de la peau et/ou du cuir chevelu, et/ou les désordres cutanés associés à la séborrhée, et/ou les désordres liés aux germes du genre *Propionibacterium,* notamment *Propionibacterium acnes et*/*ou Propionibacterium granulosum.*

L'invention concerne également un procédé de traitement cosmétique de la séborrhée et/ou des désordres cutanés qui lui sont associés, dans lequel on applique sur la peau et/ou sur le cuir chevelu, une composition cosmétique comprenant au moins un dérivé de polyaminoacide de formule (I) tel que défini ci-après.

Les désordres cutanés associés à la séborrhée peuvent notamment être la dermite séborrhéique, l'acné, la peau grasse à tendance acnéique et/ou l'hyperséborrhée.
On a en effet constaté que les dérivés de polyaminoacides selon l'invention présentaient une forte activité notamment sur *Propionibacterium* acnes et *Propionibacterium granulosum,* et pouvaient donc être utilisés dans une composition cosmétique ou pharmaceutique, notamment dermatologique, en particulier en tant qu'actif anti-séborrhéique et/ou anti-acnéique.
Les dérivés employés dans la présente invention présentent notamment comme avantage, une structure chimique clairement définie et caractérisée, d'où une reproductibilité de leur fabrication aisée; leur faisabilité industrielle est également assez simple. De plus, ils possèdent une bonne solubilité ou compatibilité avec les milieux couramment employés en cosmétique, les milieux aqueux notamment.

Les dérivés de polyaminoacides utilisés dans le cadre de la présente invention sont bien connus dans l'art antérieur, notamment dans le domaine cosmétique, en particulier pour leurs propriétés d'hydratation, ou pour leur application en capillaire. On peut notamment citer la demande FR2776510 qui décrit une composition cosmétique destinée au renforcement ou au soin des fibres kératiniques, notamment capillaire, comprenant des dérivés de polyamino-acides.

Les dérivés de polyaminoacides répondent à la formule (I) suivante : 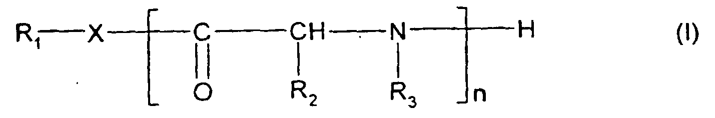 dans laquelle:
- X représente O, S, NH ou NR" avec R" représentant un radical hydrocarboné, saturé ou insaturé, linéaire ou ramifié, en C₁₋₆;
- R₁ représente
   - un radical hydrocarboné linéaire ou ramifié, saturé ou insaturé, en C₁₋₄₀, éventuellement substitué par au moins un radical hydroxy et/ou un radical -NRR' et/ou éventuellement interrompu par au moins un hétéroatome choisi parmi N, O
      ou Si, R et R', identiques ou différents, étant un atome d'hydrogène ou un radical hydrocarboné, saturé ou insaturé, linéaire ou ramifié, en C₁₋₆;
   - un radical 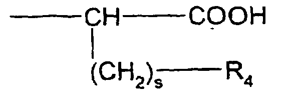 avec s égal à 0, 1, 2, 3 ou 4; et R₄ représentant un atome d'hydrogène ou un radical choisi parmi -NH₂, -OH, -SH, -CHOHCH₃, -CONH₂, -NH-C(NH₂)=NH, -C₆H₅, -C₆H₄OH et 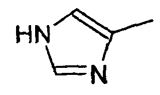
   - un radical 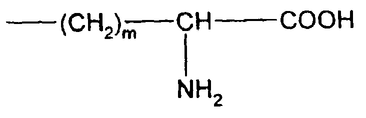 avec m égal à 3, 4 ou 5;
- R₂ représente un atome d'hydrogène; un radical hydrocarboné, saturé ou insaturé, linéaire ou ramifié, en C₁₋₈; un radical choisi parmi -CH₂C₆H₅, -CH₂C₆H₄OH,
- CH₂OH -CHOHCH₃, -(CH₂)ₜNH₂ avec t égal à 3, 4 ou 5;
- R₃ représente un atome d'hydrogène ou un radical hydrocarboné, saturé ou insaturé, linéaire ou ramifié, en C₁₋₆;
- n est un nombre moyen supérieur à 1 tel que le poids moléculaire du dérivé de polyaminoacide est compris entre 100 et 200 000;
- l'unité répétitive pouvant être identique ou différente pour un même dérivé, R₂ et/ou R₃ prenant alors au moins l'une des autres significations données pour ces radicaux, de préférence les unités sont identiques.

Les sels, notamment d'acide minéral ou d'acide organique, desdits dérivés de polyaminoacides font également partie de la présente invention.

Selon un mode de réalisation préféré de la présente invention, on emploie au moins un dérivé pour lequel l'on a une des significations suivantes :
- X représente O, S et de préférence NH ou NR" avec R" représentant un radical hydrocarboné, saturé ou insaturé, linéaire ou ramifié, en C₁₋₆;
- R₁ représente un radical hydrocarboné linéaire ou ramifié, saturé ou insaturé, en C₈₋₄₀, éventuellement substitué par au moins un radical hydroxy et/ou un radical
- NRR' et/ou éventuellement interrompu par au moins un hétéroatome choisi parmi N, O ou Si, R et R', identiques ou différents, étant un atome d'hydrogène ou un radical hydrocarboné, saturé ou insaturé, linéaire ou ramifié, en C₁₋₆;
- R₂ représente un atome d'hydrogène;
- R₃ représente un radical hydrocarboné saturé, linéaire ou ramifié, en C₁₋₆; notamment un radical méthyle ou éthyle;
- n est compris entre 2 et 100 et/ou est tel que le poids moléculaire du dérivé de polyaminoacide est compris entre 150 et 10 000.

Encore plus préférentiellement, on choisit le composé employé dans le cadre de la présente invention parmi les dérivés dans lesquels :
- X représente O, S et de préférence NH;
- R₁ représente un radical hydrocarboné, linéaire ou ramifié, saturé en C₁₀₋₂₄, éventuellement substitué par 1, 2, 3 ou 4 radicaux hydroxy ; ou un radical hydrocarboné, linéaire ou ramifié, comportant une ou plusieurs insaturations doubles, en C₁₂₋₂₄, éventuellement substitué par au moins un radical hydroxy;
- R₂ représente un atome d'hydrogène;
- R₃ représente un radical méthyle;
- n est compris entre 4 et 50 et/ou est tel que le poids moléculaire du dérivé de polyaminoacide est compris entre 300 et 8 000.

Les dérivés de polyaminoacides selon l'invention peuvent aisément être préparés par l'homme du métier sur base de ses connaissances générales. La demande FR2776510 décrit notamment un procédé permettant la préparation de ces composés.

Les dérivés de polyaminoacides peuvent être présents dans les compositions cosmétiques ou pharmaceutiques en une quantité suffisante pour obtenir l'effet recherché, et notamment comprise entre 0,001 et 30% en poids, de préférence entre 0,01 et 15% en poids, et plus particulièrement entre 0,5 et 5% en poids, par rapport au poids total de la composition.

Les compositions comprenant lesdits dérivés de polyaminoacides comprennent par ailleurs un milieu physiologiquement acceptable, notamment cosmétiquement
ou pharmaceutiquement acceptable, c'est-à-dire compatible avec la peau, les muqueuses, les cheveux et/ou le cuir chevelu.
Elles peuvent se présenter sous toutes les formes galéniques appropriées pour une application topique, notamment sous forme de solutions aqueuses, hydroalcooliques, organiques ou huileuses; de gels aqueux, hydroalcooliques ou huileux; de produits anhydres pâteux ou solides; d'émulsions eau-dans-huile, huile-dans-eau ou multiples; de suspensions ou de dispersions dans des solvants ou des corps gras, de type lotion ou sérum; de microémulsions, de microcapsules, de mi-croparticules; de dispersions vésiculaires de type ionique (liposomes) et/ou non ionique; de mousse.

Le milieu physiologiquement acceptable dans lequel les dérivés peuvent être employés, ainsi que ses constituants, leur quantité, la forme galénique de la composition et son mode de préparation, peuvent être choisis par l'homme du métier sur la base de ses connaissances générales en fonction du type de composition et d'utilisation recherchés.
Notamment, la composition peut comprendre tout corps gras usuellement utilisé dans le domaine d'application envisagé. On peut notamment citer les corps gras siliconés tels que les huiles, les gommes et les cires de silicone, ainsi que les corps gras non siliconés tels que les huiles et les cires d'origine végétale, minérale, animale et/ou synthétique. Les huiles peuvent éventuellement être volatiles
ou non volatiles. On peut encore citer les hydrocarbures, les esters et les éthers de synthèse, les alcools gras et les acides gras.
La composition peut également comprendre un milieu aqueux, un milieu hydroalcoolique contenant un alcool tel que l'éthanol ou l'isopropanol, ou un milieu organique comprenant des solvants organiques usuels tels que des alcools en C₁₋₆, notamment l'éthanol et l'isopropanol, des glycols tels que le propylène glycol, des cétones.
La composition peut comprendre au moins un émulsionnant classique, choisi parmi les émulsionnants amphotères, anioniques, cationiques ou non ioniques, utilisés seuls ou en mélange.
Elle peut également comprendre les adjuvants habituels dans le domaine considéré, tels que les épaississants ou gélifiants hydrophiles ou lipophiles, les additifs hydrophiles ou lipophiles, les actifs notamment cosmétiques, les conservateurs, les antioxydants, les parfums, les chargés, les pigments, les filtres UV, les absorbeurs d'odeur, les colorants, les hydratants (glycérine), des vitamines, des acides gras essentiels, des polymères liposolubles notamment hydrocarbonés, les opacifiants, les stabilisants, les sequestrants, les conditionneurs, les agents propulseurs.
Bien entendu l'homme du métier veillera à choisir ce ou ces éventuels adjuvants complémentaires et/ou leur quantité de manière telle que les propriétés avantageuses de la composition selon l'invention ne soient pas, ou substantiellement pas, altérées par l'adjonction envisagée.

Par exemple, lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et éventuellement le coémulsionnant peuvent être présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

Parmi les huiles utilisables, on peut citer les huiles minérales (huile de vaseline), les huiles végétales (fraction liquide de beurre de karité), les huiles animales, les huiles de synthèse (huile de purcellin, polyisobutène hydrogénée), les huiles siliconées et les huiles fluorées (perfluoropolyéthers).
Parmi les émulsionnants utilisables, on peut citer les esters d'acide gras et de polyol, tels que les esters gras de sorbitol, comme le tristéarate de sorbitan vendu sous la dénomination de Span 65 par la société ICI, ou aussi les esters gras de glycérol tels que le monostéarate de glycérol, ou le mélange palmito-stéarate de glycol / stéarate de polyéthylène glycol (6 OE) / stéarate de polyéthylène glycol (32 OE) vendu sous la dénomination « Tefose 63 » par la société Gattefosse; la lécithine hydrogénée; ou encore les esters de polyéthylèneglycol (PEG) tels que le stéarate de PEG-40 vendu sous la dénomination de Myrj 52 par la société ICI. Il peut s'agir aussi d'émulsionnants siliconés tels que le cétyl diméthicone copolyol vendu sous la dénomination d'Abil EM90 par la société Goldschmidt.
Parmi les gélifiants hydrophiles, on peut citer les gommes naturelles (gomme de xanthane), les polysaccharides (hydroxypropylmethylcellulose, carboxyméthylcellulose), les polymères carboxyvinyliques (carbomer), les copolymères acryliques tels que les copolymères d'acrylates/alkylacrylates, les poly(méth)acrylates de glycéryle tels que le produit vendu sous la dénomination de Norgel par la société Guardian, les polyacrylamides et notamment le mélange de polyacrylamide; C₁₃₋₁₄-Isoparaffine et Laureth-7, vendu sous la dénomination de Sepigel 305 par la société Seppic, les dérivés de sucres oxyéthylénés tels que le méthylglucose oxyéthyléné; comme gélifiants lipophiles, on peut citer les argiles modifiées comme les bentones, les sels métalliques d'acides gras, la silice hydrophobe et les polyéthylènes.

Parmi les actifs, hydrophiles ou lipophiles, susceptibles d'être employés dans les compositions ci-dessus, on peut citer notamment les actifs susceptibles de compléter l'effet des dérivés de polyamino-acides dans le traitement de la séborrhée et des désordres associés, et notamment de l'acné.
Il peut s'agir par exemple d'agents anti-inflammatoires tels que le peroxyde de benzoyle; d'antibiotiques tels que la clindamycine et l'érythromycine; d'agents antiseptiques tels que l'octopirox; d'actifs kératolytiques tels que l'acide salicylique et ses dérivés, les α-hydroxyacides, les β-hydroxyacides, l'acide rétinoïque et ses dérivés, le rétinol et ses dérivés; d'agents antiséborrhéiques tels que les sels de métaux di- ou trivalents comme les sels des métaux alcalino-terreux et les lanthanides.
Par ailleurs, comme actifs hydrophiles, on peut utiliser par exemple les protéines ou les hydrolysats de protéine, les acides aminés, les polyols (glycérine, propylène glycol), l'urée, l'allantoïne, les sucres et les dérivés de sucre, les vitamines hydrosolubles, l'amidon, les extraits bactériens ou végétaux, les agents hydratants.
Comme actifs lipophiles, on peut utiliser par exemple le tocophérol et ses dérivés, les acides gras essentiels, les sphingolipides, les huiles essentielles.

Lorsque la peau comportant les désordres cutanés liés à la séborrhée doit être exposée au soleil, la composition comprenant les dérivés de polyamino-acides considérés peut comprendre, en outre, au moins un filtre solaire, de façon à préserver l'efficacité du dérivé selon l'invention tout en protégeant la peau des effets néfastes des rayons solaires. Parmi les filtres solaires susceptibles d'être employés, on peut citer les pigments qui peuvent être sous forme de nanoparticules (nanopigments) ou non, et notamment les oxydes métalliques tels que titane, fer et/ou zinc. Parmi les filtres organiques, on peut citer les dérivés sulfonés ou sulfonatés de la benzophénone, les dérivés sulfoniques ou sulfonatés du benzylidène camphre et les acrylates tels que l'octocrylène. La quantité de filtre dépend de la protection solaire souhaitée et peut varier de 0,01 à 10% et de préférence de 0,1 à 5% du poids total de la composition.

Le pH des compositions selon l'invention est de préférence inférieur à 7, notamment compris entre 3 et 6.

Les dérivés selon l'invention trouvent une application toute particulière dans des compositions pouvant se présenter :
- sous la forme d'un produit de soin dermatologique ou cosmétique de la peau du visage, du corps y compris le cuir chevelu, des lèvres, tel qu'une base de soin pour les lèvres, une composition de protection solaire ou de bronzage artificiel; une composition de soin ou de traitement (de jour, de nuit, anti-rides, hydratante, etc.) pour le visage; une composition matifiante pour le visage; un gel ou une crème de nettoyage ou de démaquillage; un lait corporel de protection ou de soin; un lait ou une lotion purifiant(e); un stick camouflant;
- sous la forme d'un produit de maquillage de la peau du visage, du corps ou des lèvres, tel qu'un fond de teint, un stick anti-cernes, un stick camouflant;
- sous la forme d'un gel ou lotion après-rasage;
- sous la forme d'une composition pharmaceutique;
- sous la forme d'une composition solide telle qu'un savon ou un pain de nettoyage;
- sous la forme d'une composition pour soins capillaires, et notamment un shampooing traitant ou une lotion traitante notamment anti-séborrhéique.

Les dérivés selon l'invention trouvent une application préférée dans des compositions destinées au traitement de l'acné et/ou au traitement des peaux grasses et/ou destinées au traitement du cuir chevelu gras, donc dans des compositions dites anti-acné et/ou anti-séborrhée.

L'invention est illustrée plus en détail dans les exemples suivants.

### Exemple 1

Préparation du composé de formule (I) avec R₁ = C₈H₁₇-CH=CH-C₈H₁₆-, X = -NH-, R₂=H, R₃ = -CH₃ et n = 9,8.

Dans un réacteur de 1 litre, on met en suspension 46 g (0,4 mole) de N-carboxy-anhydride sarcosine dans 500 ml de toluène. On ajoute ensuite, goutte à goutte, 13 g (0,05 mole) d'oléylamine. Après la fin de l'addition, on porte le mélange à 80°C pendant environ 2 heures. On laisse ensuite refroidir à température ambiante puis ajoute 50 ml d'éthanol (95°C).
Après évaporation des solvants sous pression réduite et séchage sous vide, on obtient 42 g d'une poudre.
L'indice "n" a été déterminé par RMN.

En faisant varier la proportion d'oléylamine, on prépare également le dérivé de polyaminoacide ayant la même structure mais ayant un indice "n" égal à 41.

### Exemple 2

On a déterminé l'activité anti-microbienne des deux composés préparés à l'exemple 1, vis-à-vis de *Propionibacterium acnes* et *Propionibacterium granulosum.*

Les étapes pour réaliser ce test sont les suivantes :
1/ culture du microorganisme : *Propionibacterium acnes* et *Propionibacterium granulosum* sont cultivés sur gélose Tryptocaséine soja en pente;
2/ préparation de l'inoculum : 4 jours avant le début du test, on pratique un repiquage des deux souches bactériennes sur un milieu approprié et on laisse incuber pendant 4 jours à 35°C en conditions d'anaérobiose. Le jour du test, on lave la pente avec environ 9 ml de diluant. La suspension obtenue titre à 10⁸ germes/ml.
3/ préparation de l'échantillon : la veille du test, on dépose dans un flacon de verre appelé pilulier, 32 g de bouillon Tryptocaséine soja et on met à incuber à 35°C. Le jour du test, on ajoute 4 g de la composition à tester et 4 ml d'inoculum (soit 10⁷ germes/ml); on homogénéise et on place le pilulier dans un incubateur agité à 35°C. Parallèlement, on prépare un témoin pour vérifier que les germes sont dans des conditions de croissance favorables pendant la durée du test.
4/ prélèvements et dénombrements : après 24 heures de contact, on homogénéise le contenu du pilulier et on en prélève 1 g. Après avoir déterminé la dilution appropriée pour pouvoir effectuer le comptage, on étale cette dilution en surface de boîtes de Pétri gélosées (milieu Eugon LT100), puis on laisse incuber les boîtes de Pétri pendant 24 heures à l'étuve à 35°C en conditions d'anaérobiose.
   Puis, on effectue le comptage des colonies sur les boîtes contenant plus de 20 et moins de 200 colonies.

La composition testée (pH 7) comprend les constituants suivants :
- polymère carboxyvinylique 0,3 g
- eau distillée stérile 98,4 g
- triéthanolamine 0,3 g
- composé à tester 1 g

Les résultats obtenus sont indiqués dans le tableau suivant. Ils sont exprimés en nombre de micro-organismes revivifiables au bout de 24 heures, par gramme de préparation :

| | | |
|---|---|---|
| Composition | *Propionibacterium acnes* | *Propionibacterium granulosum* |
| Composition A (composé de l'ex.1, n=9,8) | 1,1.10⁶ | < 20 (seuil de sensibilité de la méthode) |
| Composition B (composé de l'ex.1, n=41) | 1,8.10⁵ | 2,2.10³ |
| Témoin | 4,4.10⁷ | 1,3.10⁸ |

On constate donc que les composés selon l'invention présentent bien une activité significative vis-à-vis de *Propionibacterium acnes et de Propionibacterium granulosum.*

Ce résultat est par ailleurs confirmé par un second test qui montre qu'après 24 heures de contact, la composition A a réduit la population initiale de *Propionibacterium granulosum* de 5,8 log₁₀, et la composition B de 4,8 log₁₀ par comparaison au placebo.

### Exemple 3 : Gel moussant pour peaux grasses séborrhéiques

- composé de l'exemple 1 (n=9,8) 1 %
- copolymère d'alcool de suif hydrogéné oxyéthyléné (60 OE) /myristyl glycol (solubilisant) (Elfacos GT 282 S de Akzo) 0,9 %
- glycérine 3%
- acide glycolique à 57% en poids dans l'eau 0,5%
- N-carboxyéthoxyéthyl N-cocoylamidoéthyl aminoacétate N-di-sodique à 38% dans l'eau 5%
- lauryl éther sulfate de sodium à 28% dans l'eau 14,3%
- chlorure de sodium 1 %
- diéthanolamide d'acides gras de coprah (adoucissant) 0,7%
- parfum qs
- mélange alcool butylique oxyéthyléné (26OE) oxypropyléné (26OP), huile de ricin hydrogénée oxyéthylénée (40OE) 1%
- eau déminéralisée qsp 100%
   Le gel obtenu convient pour le traitement de la dermite séborrhéique, en application deux fois par jour sur le visage.

### Exemple 4 : Crème nettoyante moussante pour peaux acnéiques

- monostéarate d'éthylène glycol 2%
- composé de l'exemple 1 (n=41) 0,5% silicate de magnésium et d'aluminium hydraté 1,7%
- hydroxypropylméthylcellulose 0,8%
- mélange de cocoyl isethionate de sodium et d'acides gras de coprah (Elfan AT 84 G de Akzo) 15%
- acide stéarique 1,25%
- lauroyl sarcosinate de sodium à 30 % dans l'eau 10%
- parfum qs
- eau déminéralisée qsp 100%
   La crème ainsi obtenue convient pour le nettoyage de la peau acnéique, par exemple par utilisation sur le visage deux fois par jour.

### Exemple 5 : Crème traitante pour l'acné

- tristéarate de sorbitane 1%
- composé de l'exemple 1 (n=9,8) 1,5%
- homopolymère carboxyvinylique réticulé 0,4%
- gomme de xanthane 0,5%
- copolymère diméthacrylate d'éthylèneglycol/ méthacrylate de lauryle 1 %
- cyclopentadiméthylsiloxane 6%
- glycérine 3%
- émulsionnant 4%
- parfum qs
- eau déminéralisée qsp 100%
   La crème obtenue convient pour le traitement de la peau, par application sur le visage et le dos une fois par jour.

### Exemple 6 : Gel traitant pour peaux séborrhéiques

- composé de l'exemple 1 (n=41) 1%
- gomme de xanthane 1 %
- glycérine 2%
- éthanol 20%
- mélange alcool butylique oxyéthyléné (26OE) oxypropyléné (260P), huile de ricin hydrogénée oxyéthylénée (40OE) dans l'eau 1%
- parfum qs
- eau déminéralisée qsp 100%
   Ce gel convient pour le traitement de la peau séborrhéique, en application une à deux fois par jour sur le visage.

### Exemple 7 : Crème teintée traitante pour peaux acnéiques

- lécithine hydrogénée 2,4%
- huile d'amandes d'abricot 6%
- copolymère diméthacrylate d'éthylèneglycol/méthacrylate de lauryle 1 %
- stérols de soja oxyéthylénés (5 OE) 1,6%
- composé de l'exemple 1 (n=41) 1%
- oxydes de fer 0,9%
- oxyde de titane 5%
- polyacrylamide /C₁₃-C₁₄-Isoparaffine /Laureth-7 (Sepigel 305) 4%
- cyclopentadiméthylsiloxane 6%
- glycérine 6%
- propylène glycol 6%
- parfum qs
- eau déminéralisée qsp 100%
   Cette crème est de couleur beige et convient pour le traitement de la peau, par application sur le visage deux fois par jour.

### Exemple 8 : Stick camouflant pour peaux grasses

- cires (Carnauba et Ozokérite) 14%
- fraction liquide de beurre de karité 4%
- oxydes de titane et de zinc 22%
- oxydes de fer 4%
- composé de l'exemple 1 (n=9,8) 1 %
- polydiméthylsiloxane / silice hydratée 0,1%
- alcool cétylique 1,4%
- isoparaffine qsp 100%

Le stick obtenu peut être appliqué sur le visage plusieurs fois par jour.

## Revendications

1. Utilisation d'au moins un dérivé de polyaminoacide de formule (I) : 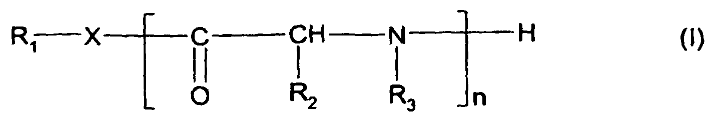 dans laquelle :
- X représente O, S, NH ou NR" avec R" représentant un radical hydrocarboné, saturé ou insaturé, linéaire ou ramifié, en C₁₋₆;
- R₁ représente
- un radical hydrocarboné linéaire ou ramifié, saturé ou insaturé, en C₁₋₄₀, éventuellement substitué par au moins un radical hydroxy et/ou un radical -NRR' et/ou éventuellement interrompu par au moins un hétéroatome choisi parmi N, O ou Si, R et R', identiques ou différents, étant un atome d'hydrogène ou un radical hydrocarboné, saturé ou insaturé, linéaire ou ramifié, en C₁₋₆;
- un radical 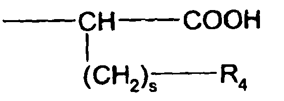 avec s égal à 0, 1, 2, 3 ou 4; et R₄ représentant un atome d'hydrogène ou un radical choisi parmi -NH₂, -OH, -SH, -CHOHCH₃, -CONH₂, -NH-C(NH₂)=NH, -C₆H₅, -C₆H₄OH et 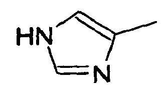
- un radical 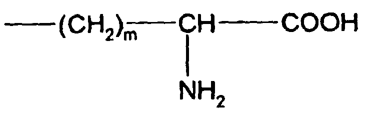 avec m égal à 3, 4 ou 5;
- R₂ représente un atome d'hydrogène; un radical hydrocarboné, saturé ou insaturé, linéaire ou ramifié, en C₁₋₈; un radical choisi parmi -CH₂C₆H₅, -CH₂C₆H₄OH,
- CH₂OH, -CHOHCH₃, -(CH₂)ₜ-NH₂ avec t égal à 3, 4 ou 5;
- R₃ représente un atome d'hydrogène ou un radical hydrocarboné, saturé ou insaturé, linéaire ou ramifié, en C₁₋₆;
- n est un nombre moyen supérieur à 1 tel que le poids moléculaire du dérivé de polyaminoacide est compris entre 100 et 200 000;
- l'unité répétitive pouvant être identique ou différente pour un même dérivé, R₂ et/ou R₃ prenant alors au moins l'une des autres significations données pour ces radicaux,
ou d'un de ses sels,
- pour traiter cosmétiquement la séborrhée de la peau et/ou du cuir chevelu, et/ou les désordres cutanés associés à la séborrhée, et/ou les désordres liés aux germes du genre *Propionibacterium,* notamment *Propionibacterium acnes* et/ou *Propionibacterium granulosum;*
ou
- pour la fabrication d'une composition pharmaceutique destinée à traiter la séborrhée de la peau et/ou du cuir chevelu, et/ou les désordres cutanés associés à la séborrhée, et/ou les désordres liés aux germes du genre *Propionibacterium,* notamment *Propionibacterium acnes* et/ou *Propionibacterium granulosum.*

2. Utilisation selon la revendication 1, pour traiter cosmétiquement la dermite séborrhéique, l'acné, la peau grasse à tendance acnéique et/ou l'hyperséborrhée

3. Utilisation selon la revendication 1, dans laquelle la composition pharmaceutique est destinée à traiter la dermite séborrhéique, l'acné, la peau grasse à tendance acnéique et/ou l'hyperséborrhée.

4. Utilisation selon l'une des revendications précédentes, dans laquelle le dérivé de polyaminoacides est de formule (I) dans laquelle :
- X représente O, S, NH ou NR" avec R" représentant un radical hydrocarboné, saturé ou insaturé, linéaire ou ramifié, en C₁₋₆;
- R₁ représente un radical hydrocarboné linéaire ou ramifié, saturé ou insaturé, en C₈₋₄₀, éventuellement substitué par au moins un radical hydroxy et/ou un radical
- NRR' et/ou éventuellement interrompu par au moins un hétéroatome choisi parmi N, O ou Si, R et R', identiques ou différents, étant un atome d'hydrogène ou un radical hydrocarboné, saturé ou insaturé, linéaire ou ramifié, en C₁₋₆;
- R₂ représente un atome d'hydrogène;
- R₃ représente un radical hydrocarboné saturé, linéaire ou ramifié, en C₁₋₆; notamment un radical méthyle ou éthyle; et/ou
- n est compris entre 2 et 100 et/ou est tel que le poids moléculaire du dérivé de polyaminoacide est compris entre 150 et 10 000.

5. Utilisation selon l'une des revendications précédentes, dans laquelle le dérivé de polyaminoacide répond à la formule (I) dans laquelle :
- X représente NH;
- R₁ représente un radical hydrocarboné, linéaire ou ramifié, saturé en C₁₀₋₂₄, éventuellement substitué par 1, 2, 3 ou 4 radicaux hydroxy ; ou un radical hydrocarboné, linéaire ou ramifié, comportant une ou plusieurs insaturations doubles, en C₁₂₋₂₄, éventuellement substitué par au moins un radical hydroxy;
- R₂ représente un atome d'hydrogène;
- R₃ représente un radical méthyle; et/ou
- n est compris entre 4 et 50 et/ou est tel que le poids moléculaire du dérivé de polyaminoacide est compris entre 300 et 8 000.

6. Utilisation selon l'une des revendications précédentes, dans laquelle le dérivé de polyaminoacides est présent dans une composition cosmétique ou pharmaceutique en une quantité comprise entre 0,001 et 30% en poids, de préférence entre 0,01 et 15% en poids, et plus particulièrement entre 0,5 et 5% en poids, par rapport au poids total de la composition.

7. Procédé de traitement cosmétique de la séborrhée et/ou des désordres cutanés qui lui sont associés, dans lequel on applique sur la peau et/ou sur le cuir chevelu, une composition cosmétique comprenant au moins un dérivé de polyaminoacide de formule (I) tel que défini à l'une des revendications 1, 4 et 5.

8. Procédé selon la revendication 7, dans lequel le dérivé de polyaminoacides est présent dans la composition cosmétique en une quantité comprise entre 0,001 et 30% en poids, de préférence entre 0,01 et 15% en poids, et plus particulièrement entre 0,5 et 5% en poids, par rapport au poids total de la composition.

9. Procédé selon l'une des revendications 7 à 8, dans lequel la composition se présente :
- sous la forme d'un produit de soin cosmétique de la peau du visage, du corps y compris le cuir chevelu, des lèvres, tel qu'une base de soin pour les lèvres, une composition de protection solaire ou de bronzage artificiel; une composition de soin ou de traitement (de jour, de nuit, anti-rides, hydratante, etc.) pour le visage; une composition matifiante pour le visage; un gel ou une crème de nettoyage ou de démaquillage; un lait corporel de protection ou de soin; un lait ou une lotion purifiant(e); un stick camouflant;
- sous la forme d'un produit de maquillage de la peau du visage, du corps ou des lèvres, tel qu'un fond de teint, un stick anti-cemes, un stick camouflant;
- sous la forme d'un gel ou lotion après-rasage;
- sous la forme d'une composition solide telle qu'un savon ou un pain de nettoyage;
- sous la forme d'une composition pour soins capillaires, et notamment un shampooing traitant ou une lotion traitante notamment anti-séborrhéique.

## Patentansprüche

1. Verwendung mindestens eines Polyaminosäurederivats der Formel (I): 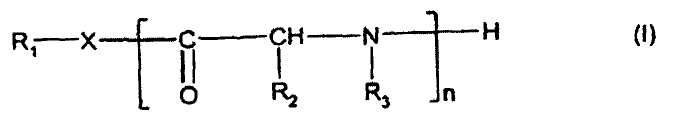 worin:
- X O, S, NH oder NR" bedeutet, wobei R" eine gesättigte oder ungesättigte, geradkettige oder verzweigte C₁₋₆₋Kohlenwasserstoffgruppe bedeutet;
- R₁ bedeutet:
- eine geradkettige oder verzweigte, gesättigte oder ungesättigte C₁₋₄₀-Kohlenwasserstoffkette, die gegebenenfalls mit mindestens einer Hydroxygruppe und/oder einer Gruppe -NRR' substituiert und/oder gegebenenfalls durch mindestens ein Heteroatom, das unter N, O oder Si ausgewählt ist, unterbrochen ist, wobei die Gruppen R und R', die gleich oder verschieden sind, ein Wasserstoffatom oder eine gesättigte oder ungesättigte, geradkettige oder verzweigte C₁₋₆₋Kohlenwasserstoffgruppe bedeuten;
- eine Gruppe 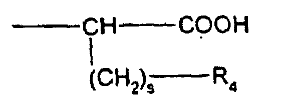 mit s = 0, 1, 2, 3 oder 4; wobei R₄ ein Wasserstoffatom oder eine Gruppe bedeutet, die ausgewählt ist unter -NH₂, -OH, -SH, -CHOHCH₃, -CONH₂, -NH-C(NH₂)=NH, -C₆H₅, -C₆H₄OH und 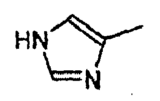
- eine Gruppe 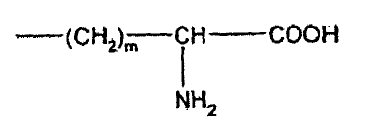 mit m = 3, 4 oder 5;
- R₂ bedeutet ein Wasserstoffatom; eine geradkettige oder verzweigte, gesättigte oder ungesättigte C₁₋₈₋Kohlenwasserstoffgruppe; eine Gruppe, die ausgewählt ist unter -CH₂C₆H₅, -CH₂C₆H₄OH, -CH₂OH, -CHOHCH₃, -(CH₂)ₜ-NH₂ mit t = 3, 4 oder 5;
- R₃ bedeutet ein Wasserstoffatom oder eine geradkettige oder verzweigte, gesättigte oder ungesättigte C₁₋₆-Kohlenwasserstoffgruppe;
- n bedeutet einen Mittelwert über 1, wobei die Zahl so gewählt ist, dass die Molmasse des Polyaminosäurederivats im Bereich von 100 bis 200.000 liegt;
- wobei die wiederkehrende Einheit in einem Derivat gleich oder verschieden sein kann und die Gruppe R₂ und/oder R₃ somit mindestens eine der anderen für diese Gruppen angegebenen Bedeutungen aufweisen, wobei die Einheiten vorzugsweise identisch sind.
oder eines Salzes dieser Verbindungen,
- zur kosmetischen Behandlung von Seborrhoe der Haut und/oder der Kopfhaut und/oder von Hautstörungen, die mit Seborrhoe verbunden sind, und/oder Störungen, die mit Keimen der Gattung *Propionibacterium,* insbesondere *Propionibacterium acnes* und/oder *Propionibacterium granulosum,* zusammenhängen; oder
- zur Herstellung einer pharmazeutischen Zusammensetzung, die für die Behandlung von Seborrhoe der Haut und/oder der Kopfhaut und/oder mit Seborrhoe zusammenhängenden Hautstörungen und/ oder Störungen vorgesehen ist, die mit Keimen der Gattung *Propionibacterium und insbesondere Propionibacterium acnes* und/oder *Propionibacterium granulosum* zusammenhängen.

2. Verwendung nach Anspruch 1 zur kosmetischen Behandlung von seborrhoischen Ekzemen, Akne, fettiger Haut mit Aknetendenz und/oder Hyperseborrhoe.

3. Verwendung nach Anspruch 1, wobei die pharmazeutische Zusammensetzung zur Behandlung von seborrhoischen Ekzemen, Akne, fettiger Haut mit Aknetendenz und/oder Hyperseborrhoe vorgesehen ist.

4. Verwendung nach einem der vorhergehenden Ansprüche, wobei das Polyaminosäurederivat die Formel (I) aufweist, worin bedeuten:
- X bedeutet O, S, NH oder NR", wobei R" eine gesättigte oder ungesättigte, geradkettige oder verzweigte C₁₋₆₋Kohlenwasserstoffgruppe ist;
- R₁ bedeutet eine geradkettige oder verzweigte, gesättigte oder ungesättigte C₈₋₄₀-Kohlenwasserstoffkette, die gegebenenfalls mit mindestens einer Hydroxygruppe und/oder einer Gruppe -NRR' substituiert und/oder gegebenenfalls durch mindestens ein Heteroatom unterbrochen ist, das unter N, O oder Si ausgewählt ist, wobei die Gruppen R und R', die gleich oder verschieden sind, Wasserstoff oder eine geradkettige oder verzweigte, gesättigte oder ungesättigte C₁₋₆-Kohlenwasserstoffgruppe bedeuten;
- R₂ bedeutet ein Wasserstoffatom;
- R₃ bedeutet eine gesättigte, geradkettige oder verzweigte C₁₋₆₋Kohlenwasserstoffgruppe, insbesondere Methyl oder Ethyl; und/oder
- n liegt im Bereich von 2 bis 100 und/oder ist so gewählt, dass die Molmasse des Polyaminosäurederivats im Bereich von 150 bis 10.000 liegt.

5. Verwendung nach einem der vorhergehenden Ansprüche, wobei das Polyaminosäurederivat der Formel (I) entspricht, worin bedeuten:
- X bedeutet NH;
- R₁ ist eine geradkettige oder verzweigte, gesättigte C₁₀₋₂₄₋Kohlenwasserstoffgruppe ist, die gegebenenfalls mit 1, 2, 3 oder 4 Hydroxygruppen substituiert ist; oder eine geradkettige oder verzweigte Kohlenwasserstoffgruppe mit einer oder mehreren Doppelbindungen und 12 bis 24 Kohlenstoffatomen, die gegebenenfalls mit mindestens einer Hydroxygruppe substituiert ist;
- R₂ bedeutet ein Wasserstoffatom;
- R₃ bedeutet die Methylgruppe; und/oder
- n liegt im Bereich von 4 bis 50 und/oder ist so gewählt, dass die Molmasse des Polyaminosäurederivats im Bereich von 300 bis 8.000 liegt.

6. Verwendung nach einem der vorhergehenden Ansprüche, wobei das Polyaminosäurederivat in einer kosmetischen oder pharmazeutischen Zusammensetzung in einem Mengenanteil von 0.001 bis 30 Gew.-%, vorzugsweise 0,01 bis 15 Gew.-% und insbesondere 0,5 bis 5 Gew.-%, bezogen auf das Gesamtgewicht der Zusammensetzung, enthalten ist.

7. Verfahren zur kosmetischen Behandlung von Seborrhoe und/oder mit Seborrhoe zusammenhängenden Hautstörungen, wobei auf die Haut und/oder die Kopfhaut eine kosmetische Zusammensetzung aufgetragen wird, die mindestens ein Polyaminsäurederivat der Formel (I), wie es in einem der Ansprüche 1, 4 und 5 definiert wurde, enthält.

8. Verfahren nach Anspruch 7, wobei das Polyaminosäurederivat in der kosmetischen Zusammensetzung in einem Mengenanteil von 0,001 bis 30 Gew.-%, vorzugsweise 0,01 bis 15 Gew.-% und insbesondere 0,5 bis 5 Gew.-%, bezogen auf das Gesamtgewicht der Zusammensetzung, enthalten ist.

9. Verfahren nach einem der Ansprüche 7 bis 8, wobei die Zusammensetzung vorliegt:
- als kosmetisches Pflegeprodukt für die Haut des Gesichts, des Körpers einschließlich der Kopfhaut, der Lippen, beispielsweise als Pflegebasis für die Lippen, Zusammensetzung zum Lichtschutz oder zur künstlichen Bräunung; Zusammensetzung zur Pflege oder Behandlung (Tagesprodukt, Nachtprodukt, Produkt gegen Falten, hydratisierendes Produkt, etc.) für das Gesicht; mattierende Zusammensetzung für das Gesicht; Gel oder Creme zur Reinigung oder zum Abschminken; Körpermilch zum Schutz oder zur Pflege; Milch oder Lotion zur Reinigung; Abdeckstift;
- als Produkt zum Schminken der Haut des Gesichts, des Körpers oder der Lippen, beispielsweise als Make-up, Stift gegen Augenringe, Abdeckstift;
- als Gel oder Lotion, die nach der Rasur angewandt werden;
- als feste Zusammensetzung, beispielsweise als Seife oder Seifenstücke;
- als Zusammensetzung für die Pflege der Haare, insbesondere als behandelndes Haarwaschmittel oder Lotion zur Behandlung insbesondere von Seborrhoe.

## Claims

1. Use of at least one polyamino acid derivative of formula (I): 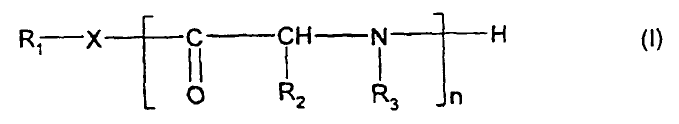 in which:
- X represents O, S, NH or NR" with R" representing a saturated or unsaturated, linear or branched C₁₋₆ hydrocarbon-based radical;
- R₁ represents
- a linear or branched, saturated or unsaturated C₁₋₄₀ hydrocarbon-based radical, optionally substituted with at least one hydroxyl radical and/or one radical - NRR' and/or optionally interrupted with at least one hetero atom chosen from N, O and Si, R and R', which may be identical or different, being a hydrogen atom or a saturated or unsaturated, linear or branched C₁₋₆ hydrocarbon-based radical;
- a radical 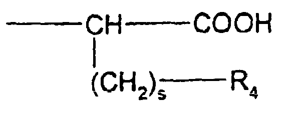 with s equal to 0, 1, 2, 3 or 4; and R₄ representing a hydrogen atom or a radical chosen from -NH₂, -OH, -SH, -CHOHCH₃, -CONH₂, -NH-C(NH₂)=NH, -C₆H₅, -C₆H₄OH and 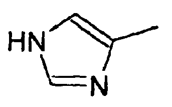
- a radical 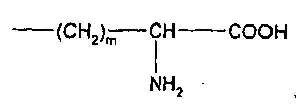 with m equal to 3, 4 or 5;
- R₂ represents a hydrogen atom; a saturated or unsaturated, linear or branched C₁₋₈ hydrocarbon-based radical; a radical chosen from -CH₂C₆H₅, -CH₂C₆H₄OH, -CH₂OH, -CHOHCH₃, -(CH₂)ₜ-NH₂ with t equal to 3, 4 or 5;
- R₃ represents a hydrogen atom or a saturated or unsaturated, linear or branched C₁₋₆ hydrocarbon-based radical;
- n is an average number greater than 1 such that the molecular weight of the polyamino acid derivative is between 100 and 200 000;
- the repeating unit possibly being identical or different for the same derivative, R₂ and/or R₃ then taking at least one of the other meanings given for these radicals,
or of one of the salts thereof,
- for the cosmetic treatment of seborrhoea of the skin and/or of the scalp, and/or of skin disorders associated with seborrhoea, and/or of disorders associated with the microorganisms of the genus *Propionibacterium,* in particular Propionibacterium *acnes* and/or *Propionibacterium granulosum;*
or
- for the manufacture of a pharmaceutical composition intended for treating seborrhoea of the skin and/or of the scalp, and/or the skin disorders associated with seborrhoea, and/or disorders associated with the microorganisms of the genus *Propionibacterium,* in particular *Propionibacterium acnes* and/or
*Propionibacterium granulosum.*

2. Use according to Claim 1, for the cosmetic treatment of seborrhoeic dermatitis, acne, greasy skin with a tendency towards acne and/or hyperseborrhoea.

3. Use according to Claim 1, in which the pharmaceutical composition is intended for treating seborrhoeic dermatitis, acne, greasy skin with a tendency towards acne and/or hyperseborrhoea.

4. Use according to one of the preceding claims, in which the polyamino acid derivative is of formula (I) in which:
- X represents O, S, NH or NR" with R" representing a saturated or unsaturated, linear or branched C₁₋₆ hydrocarbon-based radical;
- R₁ represents a linear or branched, saturated or unsaturated C₈₋₄₀ hydrocarbon-based radical, optionally substituted with at least one hydroxyl radical and/or one radical -NRR' and/or optionally interrupted with at least one hetero atom chosen from N, O and Si, R and R', which may be identical or different, being a hydrogen atom or a saturated or unsaturated, linear or branched C₁₋₆ hydrocarbon-based radical;
- R₂ represents a hydrogen atom;
- R₃ represents a saturated, linear or branched C₁₋₆ hydrocarbon-based radical; in particular a methyl or ethyl radical; and/or
- n is between 2 and 100 and/or is such that the molecular weight of the polyamino acid derivative is between 150 and 10 000.

5. Use according to one of the preceding claims, in which the polyamino acid derivative corresponds to formula (I) in which:
- X represents NH;
- R₁ represents a linear or branched, saturated C₁₀₋₂₄ hydrocarbon-based radical, optionally substituted with 1, 2, 3 or 4 hydroxyl radicals; or a linear or branched C₁₂₋₂₄ hydrocarbon-based radical comprising one or more double unsaturations, optionally substituted with at least one hydroxyl radical;
- R₂ represents a hydrogen atom;
- R₃ represents a methyl radical; and/or
- n is between 4 and 50 and/or is such that the molecular weight of the polyamino acid derivative is between 300 and 8000.

6. Use according to one of the preceding claims, in which the polyamino acid derivative is present in a cosmetic or pharmaceutical composition in an amount of between 0.001% and 30% by weight, preferably between 0.01% and 15% by weight and more particularly between 0.5% and 5% by weight, relative to the total weight of the composition.

7. Process for the cosmetic treatment of seborrhoea and/or skin disorders associated therewith, in which a cosmetic composition comprising at least one polyamino acid derivative of formula (I) as defined in one of Claims 1, 4 and 5 is applied to the skin and/or the scalp.

8. Process according to Claim 7, in which the polyamino acid derivative is present in the cosmetic composition in an amount of between 0.001% and 30% by weight, preferably between 0.01% and 15% by weight and more particularly between 0.5% and 5% by weight, relative to the total weight of the composition.

9. Process according to either of Claims 7 and 8, in which the composition is:
- in the form of a cosmetic skincare product for the face, the body including the scalp, or the lips, such as a care base for the lips, an antisun protective composition or an artificial tanning composition; a care or treatment composition (day product, night product, anti-wrinkle product, moisturizer, etc.) for the face; a matt-effect composition for the face; a cleansing or make-up-removing gel or cream; a protective body milk or a bodycare milk; a purifying milk or lotion; a cover stick;
- in the form of a make-up product for the skin of the face, the body or the lips, such as a foundation, a concealer stick or a cover stick;
- in the form of an aftershave gel or lotion;
- in the form of a solid composition such as a cleansing soap or cleansing bar;
- in the form of a haircare composition, and in particular a medicated shampoo or a medicated lotion, in particular one which is anti-seborrhoeic.
